# EUROPÄISCHE PATENTANMELDUNG

(11) **EP 0 606 050 A1**
(43) Veröffentlichungstag der Anmeldung: **13.07.1994**
(21) Anmeldenummer: 93810911.3
(22) Anmeldetag: 27.12.1993
(51) Int. Cl.: A61F 13/26

(54) **Mit einem Halter versehener Tampon**

(30) Priorität: 05.01.1993 CH 22/93
(71) Anmelder: Plüss, Bruno, CH-4310 Rheinfelden (CH)
(72) Erfinder: Plüss, Bruno, CH-4310 Rheinfelden (CH)
(74) Vertreter: Eder, Carl E.

(57) **Zusammenfassung**

Die als Halter für das Einführen des mit einer Schnur (2) versehenen Tampons (1) in die Vagina dienende Hülse (3) weist in der Nähe desjenigen Endes, aus dem der Tampon ausgestossen wird, ein Loch (3b) auf, durch welches die Ausziehschnur (2) hindurchgeführt ist. Am andern Ende weist die Hülse (3) zwei Schlitze (3c, 3d) sowie eine ringsherum laufende Rippe (3e) auf, sodass das freie Ende der Schnur quer über die Hülse (3) gespannt und um sie herum geführt werden kann, damit sie sich beim Fassen der Hülse sicher und ohne Schwierigkeit festhalten lässt. Dadurch wird der Tampon (1), wenn er nur noch mit einem kurzen Abschnitt in der Hülse (3) sitzt, sicher festgehalten, sodass er sich ohne Schwierigkeit in die Vagina einführen lässt.

## Beschreibung

Die vorliegende Erfindung betrifft einen mit einem Halter versehenen, zum Einsetzen in die Vagina bestimmten Tampon. Es ist eine derartige Tampon/Halter Kombination bekannt, bei welcher der Halter als zylindrische, den Tampon umschliessende, steife Hülse ausgebildet und der Tampon so mit einer aus der Hülse herausgeführten Ausziehschnur versehen ist, dass er sich durch Ziehen an der Schnur teilweise aus der Hülse ausschieben lässt. Bei dieser bekannten Ausführungsform ist die den Tampon umgebende, als Halter dienende Hülse etwas, also beispielsweise 30 - 60%, länger als der Tampon, sodass der in ihrer Mitte untergebrachte Tampon gut geschützt ist. Die Hülse weist an ihrem vordern Ende, also am Austossende, einen 7 - 13 mm tiefen Schlitz auf. Der Tampon ist an seinem einen, beim Einführen in die Vagina vordern Ende nicht gerade abgeschnitten, sondern etwas verjüngt, beispielsweise halbkugelig ausgebildet, während das gegenüberliegende, also das hintere Ende eben ausgebildet ist. An diesem hinteren Ende ist die Ausziehschnur befestigt. Sie ist vom hinteren Tamponende weg neben dem Tampon vorbei zum Schlitz am vorderen Hülsenende geführt und kommt dort aus der Hülse heraus, sodass durch Ziehen an dieser Schnur der Tampon aus der Hülse ausgeschoben werden kann. Mit dem Schlitz wird bezweckt, dass dann, wenn die Schnur zum hinteren Hülsenende hin gezogen wird, der Tampon mit seinem Ende, dessen Länge der Schlitzlänge entspricht, in der Hülse bleibt, sodass diese ihm als Halter zum Einführen in die Vagina dient.

Diese Ausführungsform weist nun verschiedene, nicht unwesentliche Nachteile auf: Einerseits wird durch den Schlitz die Festigkeit der Hülse reduziert, sodass sie eine Wandstärke aufweisen muss, die grösser ist, als das für eine schlitzlose Hülse nötig wäre. Andererseits, wird beim unsorgfältigen Ziehen an der Schnur, nämlich dann, wenn der Zug nicht sorgfältig zum hinteren Hülsenende hin erfolgt, der Tampon ganz aus der Hülse herausgezogen, sodass er mühsam wieder eingesetzt werden muss, und überdies muss beim Einführen des Tampons in die Vagina die Schnur mühsam festgehalten werden, damit der Tampon nicht in die Hülse zurückgeschoben wird. Des weiteren können die vordern Schlitzenden beim Einführen in die Vagina Verletzungen verursachen.

Der Behebung all dieser Nachteile dient nun die vorliegende, neue Kombination von Tampon und Hülse, bei welcher sich die Hülse von der bekannten Hülse dadurch unterscheidet, dass sie einerseits in der Nähe desjenigen Endes, aus dem der Tampon auszuschieben ist, ein Loch aufweist, durch welches die Schnur hinausgeführt ist, und dass sie andererseits am andern Ende zwei Schlitze aufweist und vorzugsweise auf ihrer Aussenfläche mit einer ringsherum laufenden Rippe versehen ist.

Nachfolgend wird anhand der beiliegenden Zeichnung ein Ausführungsbeispiel der Erfindung beschrieben. In der Zeichnung zeigt
die Fig. 1 einen in der Hülse untergebrachten Tampon, also so, wie er im Handel erhältlich sein wird, wobei allerdings die aus hygienischen Gründen übliche Schutzhülle weggelassen ist, während
die Fig. 2 die aus Hülse und Tampon bestehende Vorrichtung in der Stellung zeigt, wie sie zum Einsetzen des Tampons in die Vagina benutzt wird.

Der als Ganzes mit 1 bezeichnete Tampon weist ein abgerundetes vorderes Ende 1a und ein ebenes hinteres Ende 1b auf. In der Mitte dieser hinteren Abschlussfläche 1b ist die Ausziehschnur 2 befestigt. Dieser Tampon sitzt ungefähr in der Mitte einer vorzugsweise aus Kunststoff oder einem biologisch abbaubaren Material (wie z.B. Stärke) bestehenden, steifen, zylindrischen Hülse 3, die ca. 30% bis 60% länger ist als der Tampon 1 und deren Durchmesser so bemessen ist, dass der an sich etwas elastische Tampon nicht ohne weiteres herausrutschen, aber ohne allzugrossen Kraftaufwand herausgezogen werden kann, wie das aus der Literatur bekannt ist.

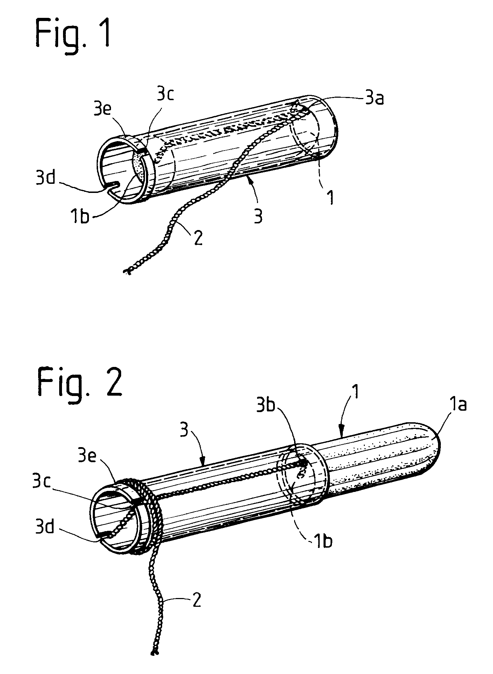

Neu ist nun die spezielle Ausgestaltung der Hülse 3: Sie besitzt in der Nähe des Ausstossendes 3a ein Loch 3b, aus welchem die Ausziehschnur 2 herausgeführt ist. Am andern Ende weist sie zwei Schlitze, Kerben oder Einschnitte 3c und 3d auf sowie eine ringsherum laufende Rippe 3e. Es ist besonders zweckmässig, wenn die Hülse am Ausstossende eine leichte, in der Zeichnung kaum feststellbare konische Verengung aufweist. Wenn man den Tampon in die Vagina einführen will, wird man ihn durch Ziehen an der Schnur 2 von der in der Fig. 1 dargestellten Lage in die in der Fig. 2 dargestellten Lage bringen. Da die Hülse 3 in der Nähe ihres Ausstossendes aber in einem ca. 5 - 12 mm betragenden Abstand von diesem Ende ein Loch 3b aufweist, durch welches die Schnur 2 nach Aussen geführt ist, ist es nicht möglich, den Tampon durch Ziehen an der Schnur ganz aus der Hülse herauszuziehen. Der Tampon bleibt also mit seinem hinteren Ende in der Hülse 2 sitzen, ohne dass es nötig ist, zu diesem Zweck besonders auf die Zugrichtung zu achten. Falls, wie das bei einer bevorzugten Ausführungsform der Fall ist, das vordere Hülsenende ganz leicht konisch verengt ist, wird auch dann ein hinreichend sicherer Sitz des Tampons gewährleistet, wenn dieser nur mit seinem 5 - 10 mm langen Ende in der Hülse sitzt. Abgesehen davon wird durch jeden Zug an der Schnur 2 der Tampon in der in der Fig. 2 dargestellten Lage festgehalten. Dadurch, dass die Hülse am Ausstossende keine Schlitze aufweist, entfällt auch die Möglichkeit der Erzeugung von Verletzungen beim Einführen.

Zum mühelosen Festhalten der Schnur 2 und damit zur Aufrechterhaltung einer sicheren Verbindung zwischen Tampon und Halter beim Einführen des Tampons in die Vagina dienen nun die beiden Schlitze 3c und 3d im Zusammenwirken mit der ringsherumlaufenden Rippe 3e. Wie aus der Fig. 2 ohne weiteres ersichtlich ist, wird nach dem durch das Ziehen an der Schnur 2 bewirkte Ausstossen des Tampons aus der Hülse 3 das freie Ende der Schnur 2 in die beiden Schlitze 3c und 3d gelegt und dann mindestens einmal um die Hülse herum geführt, wobei die Rippe das Abrutschen der Schnur verhindert. So wird man beim Halten der Hülse auch ohne besondere Aufmerksamkeit die Schnur festhalten und hat damit die Möglichkeit, den Tampon ohne Schwierigkeit sicher in die Vagina einzuführen. Wenn der Tampon eingeführt ist, kann man die Schnur los lassen und die Hülse wegnehmen, während der Tampon in der Vagina bleibt.

## Patentansprüche

1. Mit einem Halter versehener, zum Einsetzen in die Vagina bestimmter Tampon (1), wobei der Halter als zylindrische, den Tampon umschliessende, steife Hülse (3) ausgebildet und der Tampon (1) so mit einer aus der Hülse heraus geführten Ausziehschnur (2) versehen ist, dass er sich durch Ziehen an der Schnur teilweise aus der Hülse (3) ausschieben lässt, dadurch gekennzeichnet, dass die Hülse (3) in der Nähe desjenigen Endes (3a), aus dem der Tampon auszuschieben ist, ein Loch (3b), durch welches die Schnur (2) nach aussen geführt ist, und am anderen Ende zwei Schlitze (3c, 3d) aufweist.

2. Tampon mit Halter nach Anspruch 1, dadurch gekennzeichnet, dass die Hülse (3) an dem Ende (3a), aus dem der Tampon auszuschieben ist, eine konische Verengung aufweist.

3. Tampon mit Halter nach einem der Ansprüche 1 oder 2, dadurch gekennzeichnet, dass die Hülse (3) an dem mit den beiden Schlitzen (3c, 3d) versehenen Ende auf ihrer Aussenfläche eine ringsherum laufende Rippe (3e) aufweist.
